# EUROPÄISCHE PATENTSCHRIFT

(11) **EP 3 634 199 B1**
(45) Veröffentlichungstag und Bekanntmachung des Hinweises auf die Patenterteilung: **01.03.2023**
(21) Anmeldenummer: 18731004.0
(22) Anmeldetag: 04.06.2018
(51) Int. Cl.: A61B 1/00, A61B 1/12

(54) **VERFAHREN ZUM AUFBEREITEN EINES ENDOSKOPS IN EINEM AUFBEREITUNGSGERÄT UND AUFBEREITUNGSGERÄT**
METHOD FOR STERILISING AN ENDOSCOPE IN A STERILISATION DEVICE AND STERILISATION DEVICE
PROCÉDÉ DE PRÉPARATION D'UN ENDOSCOPE DANS UN APPAREIL DE PRÉPARATION ET APPAREIL DE PRÉPARATION

(30) Priorität: 03.07.2017 DE 102017114816
(43) Veröffentlichungstag der Anmeldung: 15.04.2020
(73) Patentinhaber: Olympus Winter&Ibe GmbH, 22045 Hamburg (DE)
(72) Erfinder: ANDRADE, Jan Batista, 22087 Hamburg (DE)
(74) Vertreter: Noack, Andreas
(86) Internationale Anmeldenummer: PCT/EP2018/064670
(87) Internationale Veröffentlichungsnummer: WO 2019/007608

(56) Entgegenhaltungen:
- DE-A1-102013 223 375
- DE-B3-102016 200 037
- US-A1- 2001 032 494
- US-A1- 2017 020 367

## Beschreibung

Die Erfindung betrifft ein Verfahren zum Aufbereiten eines Endoskops in einem Aufbereitungsgerät, mit den Schritten: Verbinden eines Innenraums des Endoskops mit einem Dichtigkeitstester des Aufbereitungsgeräts, Beaufschlagen des Innenraums mit einem Fluid unter einem vorgegebenen Druck, und Durchführen eines Aufbereitungsprozesses unter Einsatz von Spülflüssigkeit, wobei der Druck in dem Innenraum des Endoskops während des Aufbereitungsprozesses überwacht wird, und wobei das aufzubereitende Endoskop als fehlerhaft erkannt wird, wenn der Verlauf des Drucks von einem vorgebbaren oder vorgegebenen Druckverlauf abweicht.

Die Erfindung betrifft zusätzlich ein Aufbereitungsgerät für Endoskope.

Endoskope werden seitlängerer Zeit insbesondere in der Medizin eingesetzt, um schwer zugängliche Bereiche im Körper eines menschlichen oder tierischen Patienten zu untersuchen und/oder zu behandeln. Dazu weisen Endoskope zumeist einen langgestreckten Schaft auf, an dessen distalen Ende sich ein Objektiv befindet, durch welches ein interessierender Bereich im Körper des Patienten betrachtet werden kann. Am proximalen Ende des Schaftes befindet sich zumeist ein Hauptkörper, welcher zur Handhabung des Endoskops dient.

Endoskope weisen oftmals innere Kanäle auf, durch welche Fluide an das distale Ende des Endoskops gefördert werden können, um dort abgegeben zu werden. Alternativ können durch entsprechende Kanäle auch Fluide am distalen Ende des Endoskops aufgenommen und in Richtung des Hauptkörpers abgesaugt werden. Weiterhin können Behandlungsinstrumente durch entsprechende Kanäle des Endoskops zum distalen Ende vorgeschoben werden, um einen Eingriff an dem interessierenden Bereich des Körpers vorzunehmen.

Endoskope sind in der Herstellung sehr aufwendig und daher teuer. Um Kosten zu sparen werden Endoskope daher mehrfach verwendet. Dies erfordert eine gründliche Aufbereitung der Endoskope zwischen zwei Anwendungen, um die Übertragung von Krankheiten zu verhindern.

Grundsätzlich wird zwischen starren und flexiblen Endoskopen unterschieden. Starre Endoskope weisen in der Regel einen Schaft und Kanäle aus einem medizinischen Edelstahl auf und sind weitestgehend oder vollständig mittels stoffschlüssiger Verbindungen abgedichtet. Daher können starre Endoskope problemlos aufbereitet werden.

Flexible Endoskope weisen hingegen zumindest im Bereich des Schafts eine Hülle sowie Kanäle aus einem flexiblen Kunststoff auf. Wird dieser Kunststoff beschädigt, so kann während der Verwendung Schmutz in den Innenraum des Endoskops eindringen, welcher sich während der Aufbereitung kaum wirksam entfernen lässt. Ebenso kann während der Aufbereitung, welche zumeist unter Einsatz verschiedener Spülflüssigkeiten erfolgt, Spülflüssigkeit in den Innenraum des Endoskops eindringen und dies nachhaltig schädigen.

Es ist daher vorgeschrieben, vor der Aufbereitung flexibler Endoskope zu prüfen, ob die Hülle des Endoskops intakt ist. Dazu weisen moderne Aufbereitungsgeräte einen Dichtigkeitstester auf.

Der Dichtigkeitstester funktioniert nach dem Prinzip, dass der Innenraum des Endoskops über einen speziell vorgesehenen Anschluss mit einem Fluid, zumeist Luft, beaufschlagt wird. Dann wird der Druckverlauf in dem Innenraum gemessen und aus dem Druckverlauf wird geschlossen, ob das Endoskop hinreichend dicht ist.

Der Druck in dem Innenraum des Endoskops wird zumeist während der eigentlichen Aufbereitung aufrechterhalten, so dass auch durch unerkannte Mikrodefekte in der Kunststoffhülle keine Spülflüssigkeit in den Innenraum eindringen kann.

Bei der Aufbereitung flexibler Endoskope werden nacheinander unterschiedliche Spülflüssigkeiten bei unterschiedlichen Temperaturen eingesetzt. Dabei steigt auch die Temperatur des Fluids im Innenraum des Endoskops an. Um eine Schädigung der Kunststoffhülle des Endoskops durch zu hohen Innendruck zu vermeiden, wird der Druck während des Aufbereitungsvorgangs überwacht. Übersteigt der Druck einen oberen Grenzwert, so wird etwas von dem Fluid abgelassen, um den Druck zu senken.

Sinkt die Temperatur wieder, so fällt auch der Druck im Innenraum des Endoskops. Sobald der Druck unter einen unteren Grenzwert abfällt, wird wieder zusätzliches Fluid in den Innenraum gefördert, um den Druck wieder auf den Sollwert zu bringen.

Der Dichtigkeitstester überwacht wie lange es dauert, bis nach einem Druckabfall wieder der Sollwert des Drucks erreicht wird. Dauert dies zu lange, so wird das Endoskop als defekt erkannt und der Aufbereitungsprozess wird abgebrochen.

Ein Abfall des Drucks im Innenraum des Endoskops kann ebenfalls durch eine Undichtigkeit der Kunststoffhülle verursacht werden. Der Dichtigkeitstester überwacht daher ebenfalls, wie oft der Druck unter den unteren Grenzwert abfällt. Geschieht dies zu oft, wird das Endoskop ebenfalls als defekt erkannt.

Die Grenzwerte, bei welchen der Dichtigkeitstester ein Endoskop als defekt erkennt, weisen in der Regel eine gewisse Toleranz auf, dadurch sollen Fehlerkennungen vermieden werden. Durch diese Toleranz wird jedoch die Fähigkeit des Dichtigkeitstesters, auch geringe Undichtigkeiten aufzuspüren, eingeschränkt. Ein Verfahren zur Aufbereitung eines Endoskops gemäß dem Oberbegriff des unabhängigen Anspruchs 1 ist aus der Druckschrift DE 10 2016 200037 B3 bekannt.

Es besteht daher die Aufgabe der Erfindung darin, ein Verfahren zur Aufbereitung von Endoskopen in einem Aufbereitungsgerät, und ein entsprechendes Aufbereitungsgerät, bereitzustellen, welches hinsichtlich der beschriebenen Problematik verbessert ist.

Diese Aufgabe wird gemäß der vorliegenden Erfindung im unabhängigem Anspruch 1 gelöst durch ein Verfahren zum Aufbereiten eines Endoskops in einem Aufbereitungsgerät, mit den Schritten: Verbinden eines Innenraums des Endoskops mit einem Dichtigkeitstester des Aufbereitungsgeräts, Beaufschlagen des Innenraums mit einem Fluid unter einem vorgegebenen Druck, und Durchführen eines Aufbereitungsprozesses unter Einsatz von Spülflüssigkeit, wobei der Druck in dem Innenraum des Endoskops während des Aufbereitungsprozesses überwacht wird, und wobei das aufzubereitende Endoskop als fehlerhaft erkannt wird, wenn der Verlauf des Drucks von einem vorgebbaren oder vorgegebenen Druckverlauf abweicht, wobei das Verfahren dadurch weitergebildet ist, dass der vorgegebene oder vorgebbare Druckverlauf in Abhängigkeit von Prozessparametern des Aufbereitungsprozesses festgelegt wird.

Durch die beschriebene Maßnahme können Schwankungen des Druckverlaufs, welche durch Prozessparameter des Aufbereitungsprozesses verursacht sind, von solchen Schwankungen des Druckverlaufs unterschieden werden, welche durch Undichtigkeiten des Endoskops verursacht sind. Dadurch kann die Fähigkeit des Dichtigkeitstesters, Undichtigkeiten des Endoskops zu erkennen, verbessert werden.

In einer Weiterbildung eines Verfahrens nach der Erfindung kann Fluid aus dem Innenraum des Endoskops abgelassen werden, wenn der Druck einen oberen Grenzwert übersteigt. Dadurch wird verhindert, dass das Endoskop durch einen zu hohen Druck in seinem Innenraum beschädigt wird, wenn der Druck beispielsweise durch eine Temperaturerhöhung ansteigt.

Zusätzlich oder alternativ kann zusätzliches Fluid in den Innenraum des Endoskops eingebracht werden, wenn der Druck einen unteren Grenzwert unterschreitet. Hierdurch ist sichergestellt, dass auch bei einem Druckabfall, beispielsweise nach einem Absinken der Temperatur, ein ausreichender Druck in dem Innenraum des Endoskops aufrechterhalten wird. Dadurch kann ein Eindringen von Spülflüssigkeit in den Innenraum des Endoskops durch mikroskopische Undichtigkeiten des Endoskops vermieden werden.

In einer besonderen möglichen Ausgestaltung eines Verfahrens nach der Erfindung ist der vorgegebene oder vorgebbare Druckverlauf durch eine Zeit charakterisiert, welche maximal benötigt werden darf, um den Druck nach Unterschreiten des unteren Grenzwerts auf einen Sollwert zu bringen. Wird durch den Dichtigkeitstester zusätzliches Fluid in den Innenraum des Endoskops eingebracht, so muss der Druck entsprechend ansteigen und innerhalb einer vorgegebenen Zeit einen Sollwert erreichen. Erreicht der Druck den Sollwert jedoch innerhalb einer vorgegebenen Zeit nicht, so deutet dies darauf hin, dass eine Undichtigkeit des Endoskops vorliegt.

Gemäß einer weiteren möglichen Ausgestaltung eines Verfahrens nach der Erfindung ist der vorgegebene oder vorgebbare Druckverlauf durch eine maximale Häufigkeit charakterisiert, mit welcher der Druck den unteren Grenzwert unterschreiten darf. Ein langsames Abfallen des Drucks durch systemimmanente Undichtigkeiten ist technisch nicht vollständig vermeidbar. Bei bekannter Laufzeit eines Aufbereitungsprozesses und einer zulässigen Druckabfallrate kann ermittelt werden, wie oft der Druck während des Aufbereitungsprozesses den unteren Grenzwert unterschreiten darf. Wird der untere Grenzwert während des Aufbereitungsprozesses häufiger unterschritten, so ist dies ein Indiz dafür, dass eine unzulässige Undichtigkeit des Endoskops vorliegt.

In einer bevorzugten Variante eines Verfahrens nach der Erfindung können die Prozessparameter die Temperatur der Spülflüssigkeit umfassen. Währen des Aufbereitungsprozesses kontaktiert und durchströmt die Spülflüssigkeit das Endoskop und beeinflusst dadurch die Temperatur des Fluids in Innenraum des Endoskops, und somit auch dessen Druck. Durch Berücksichtigung der Temperatur der Spülflüssigkeit bei der Bestimmung eines zulässigen Druckverlaufs können temperaturbedingte Druckschwankungen besser von Druckschwankungen durch Undichtigkeiten unterschieden werden.

Besonders bevorzugt können die Prozessparameter den Verlauf der Temperatur der Spülflüssigkeit umfassen. Hierdurch kann die Empfindlichkeit bei der Erkennung von Undichtigkeiten weiter erhöht werden.

In einer möglichen Weiterbildung eines Verfahrens nach der Erfindung kann die Temperatur bzw. der Temperaturverlauf der Spülflüssigkeit mittels in dem Aufbereitungsgerät vorgesehener Sensoren bestimmt werden. Solche Sensoren sind in dem Aufbereitungsgerät ohnehin vorhanden, um die Temperatur der Spülflüssigkeit entsprechend der Anforderungen des Aufbereitungsprozesses zu steuern.

Alternativ oder zusätzlich kann die Temperatur bzw. der Temperaturverlauf der Spülflüssigkeit anhand einer in einer Steuerung des Aufbereitungsgeräts hinterlegten Solltemperatur bzw. eines Solltemperaturverlaufs bestimmt werden. Hierdurch kann die während des Aufbereitungsprozesses erforderliche Datenkommunikation zwischen der Steuerung und dem Dichtigkeitstester reduziert werden, da der zulässige Druckverlauf schon zu Beginn des Aufbereitungsprozesses ermittelt werden kann, ohne auf Echtzeitdaten von Sensoren zugreifen zu müssen.

In einer möglichen besonderen Ausgestaltung eines Verfahrens nach der Erfindung sind in der Steuerung des Aufbereitungsgeräts mehrere Aufbereitungsprogramme für unterschiedliche Endoskoptypen und/oder unterschiedliche Arten und/oder Intensitäten der Verschmutzung hinterlegt, zu Beginn der Aufbereitung des Endoskops wird ein Aufbereitungsprogramm ausgewählt, und ein zu dem ausgewählten Aufbereitungsprogramm hinterlegter Temperaturverlauf der Spülflüssigkeit wird zur Festlegung des vorgegebenen oder vorgebbaren Druckverlaufs verwendet. Dadurch kann die Erkennung von Undichtigkeiten individuell für den jeweils durchzuführenden Aufbereitungsprozess optimal erfolgen.

Gemäß der vorliegenden Erfindung im unabhängigen Anspruch 11 wird die Aufgabe gelöst durch ein Aufbereitungsgerät für Endoskope, mit einem Aufbereitungsaggregat, einer Steuerung, und einem Dichtigkeitstester, welches eingerichtet ist, ein Verfahren gemäß der obigen Beschreibung auszuführen. Bezüglich der dadurch erzielten Wirkungen und Vorteile wird explizit auf das oben gesagte verwiesen.

Die Erfindung wird nachfolgend anhand einiger beispielhafter Figuren näher erläutert. Es zeigen:
Fig.1: Den prinzipiellen Aufbau eines Aufbereitungsgeräts für Endoskope,
Fig.2: den prinzipiellen Ablauf eines Aufbereitungsprozesses,
Fig.3: einen möglichen Verlauf der Temperatur und des Innendrucks während eines Aufbereitungsprozesses eines intakten Endoskops,
Fig.4: einen möglichen Verlauf der Temperatur und des Innendrucks während eines Aufbereitungsprozesses eines defekten Endoskops.

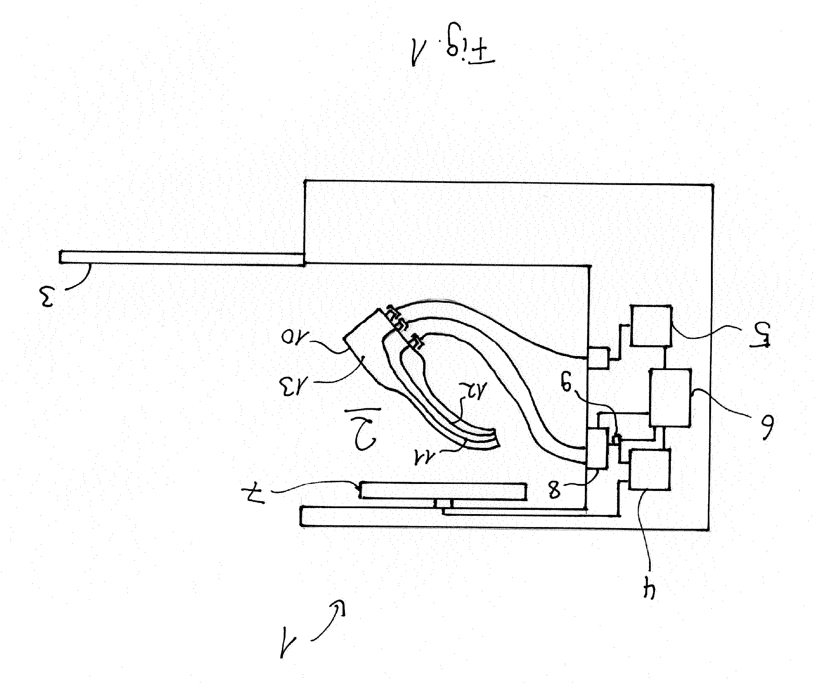

In Figur 1 ist der prinzipielle Aufbau eines Aufbereitungsgeräts für Endoskope dargestellt. Das Aufbereitungsgerät 1 weist einen Spülraum 2 auf, welcher durch eine Tür 3 verschlossen werden kann. Das Aufbereitungsgerät 1 umfasst weiterhin eine Spüleinrichtung 4, einen Dichtigkeitstester 5 sowie eine Steuerung 6.

Die Spüleinrichtung 4 ist mit einem Sprüharm 7 in dem Spülraum 2 verbunden. Weiterhin ist die Spüleinrichtung 4 mit einem Spülverteiler 8 verbunden. An einer von der Spüleinrichtung 4 zum Spülverteiler 8 verlaufenden Leitung ist ein Temperaturfühler 9 angeordnet. Spüleinrichtung 4, Sprüharm 7 und Spülverteiler 8 bilden ein Aufbereitungsaggregat des Aufbereitungsgeräts 1.

In dem Spülraum 2 ist ein aufzubereitendes Endoskop 10 angeordnet. Das Endoskop weist zwei innere Kanäle 11,12 sowie einen Innenraum 13 auf. Zur Aufbereitung sind die Kanäle 11,12 des Endoskops 10 über Schläuche mit dem Spülverteiler 8 verbunden. Der innenraum 13 des Endoskops 10 ist über einen weiteren Schlauch mit dem Dichtigkeitstester 5 verbunden.

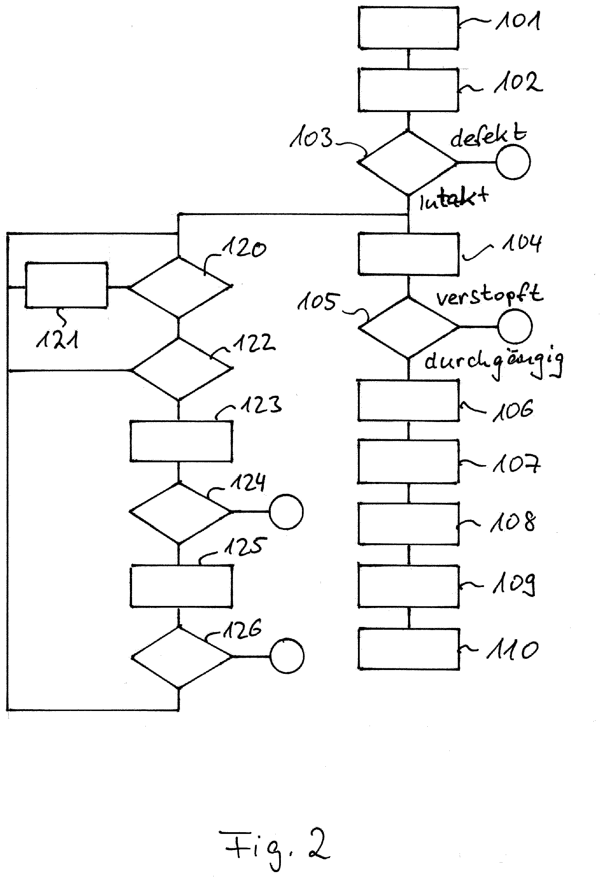

In Figur 2 ist der Ablauf eines Aufbereitungsprozesses für ein Endoskop dargestellt.

In einem Schritt 101 wird das Endoskop 10 mit dem Spülverteiler 8 und dem Dichtigkeitstester 5 verbunden, anschließend wird die Tür 3 verschlossen.

In einem nächsten Schritt 102 wird ein initialer Dichtigkeitstest des Endoskops 10 durchgeführt. Dazu wird der Innenraum 13 des Endoskops 10 durch den Dichtigkeitstester 5 mit einem Fluid, beispielsweise Luft, gefüllt, bis ein vorgegebener Solldruck erreicht ist. Anschließend wird der Druck für eine vorgegebene Zeit überwacht. Wird der Solldruck nicht in einer vorgegebenen Zeit erreicht, oder fällt der Druck anschließend unter eine untere Grenze ab, so wird das Endoskop 10 als defekt erkannt, und der Prozess wird abgebrochen (Verzweigung 103).

Als nächstes wird in einem Schritt 104 die Durchgängigkeit der Kanäle 11,12 überprüft. Dazu aktiviert die Steuerung 6 die Spüleinrichtung 4, um Spülflüssigkeit, beispielsweise Wasser, zum Spülverteiler 8 zu pumpen. Der Spülverteiler 8 ist mit nicht dargestellten Ventilen versehen, die von der Steuerung 6 so angesteuert werden, dass die Spülflüssigkeit nacheinander durch die Kanäle 11,12 geleitet wird. Dabei prüft die Spüleinrichtung 4 den Durchfluss durch die Kanäle 11,12. Wenn der Durchfluss durch einen der Kanäle 11,12 unzureichend ist, wird das Endoskop 10 als verstopft erkannt und der Prozess wird abgebrochen (Verzweigung 105).

Sind die Kanäle 11,12 des Endoskops 10 durchgängig, so wird das Endoskop 10 in einem Schritt 106 mit Reinigungsmittel behandelt. Dazu pumpt die Spüleinrichtung 4 Reinigungsmittel zu dem Spülverteiler 8 und zu dem Sprüharm 7, so dass die Kanäle 11,12 von innen und das Endoskop 10 von außen mit Reinigungsmittel durchströmt bzw. benetzt werden.

In einem nächsten Schritt 107 werden Reste des Reinigungsmittels entfernt, indem die Kanäle 11,12 von innen und das Endoskop 10 von außen mit Wasser gespült werden.

In einem folgenden Prozessschritt 108 werden die Kanäle 11,12 des Endoskops 10 von innen und das Endoskop 10 von außen mit einem Desinfektionsmittel behandelt.

In einem Schritt 109 werden wiederum Reste des Desinfektionsmittels mit Wasser abgespült. In einem letzten Prozessschritt 110 wird das Endoskop 10 getrocknet.

Parallel zu den Prozessschritten 104 bis 110 läuft als Schleife eine ständige Überwachung des Drucks in dem Innenraum 13 des Endoskops 10. Dazu wird zunächst geprüft, ob der Druck eine zulässige Obergrenze überschritten hat (Verzweigung 120). In diesem Fall wird in Schritt 121 Fluid abgelassen, bis der Druck wieder dem Sollwert entspricht. Danach beginnt die Schleife von neuem.

Ist der Druck nicht zu hoch, so wird geprüft, oder der Druck unter einen unteren Grenzwert abgefallen ist (Verzweigung 122). Ist dies nicht der Fall, so beginnt die Schleife von neuem. Ist der Druck hingegen unter den unteren Grenzwert abgefallen, so wird in Schritt 123 ein Zähler erhöht, welcher die Anzahl der Druckabfälle anzeigt. Erreicht dieser Zähler einen vorgegebenen Höchstwert, so wird das Endoskop 10 als defekt erkannt und der Aufbereitungsprozess wird abgebrochen (Verzweigung 124).

Ist der Höchstwert noch nicht erreicht, so wird in Schritt 125 erneut Fluid in des Innenraum 13 des Endoskops 10 eingebracht, um den Druck wieder auf den Solldruck zu erhöhen. Wird der Solldruck nicht in einer vorgegebenen Zeit erreicht, so wird das Endoskop 10 ebenfalls als defekt erkannt und der Aufbereitungsprozess abgebrochen (Verzweigung 126). Sonst beginnt die Schleife von neuem.

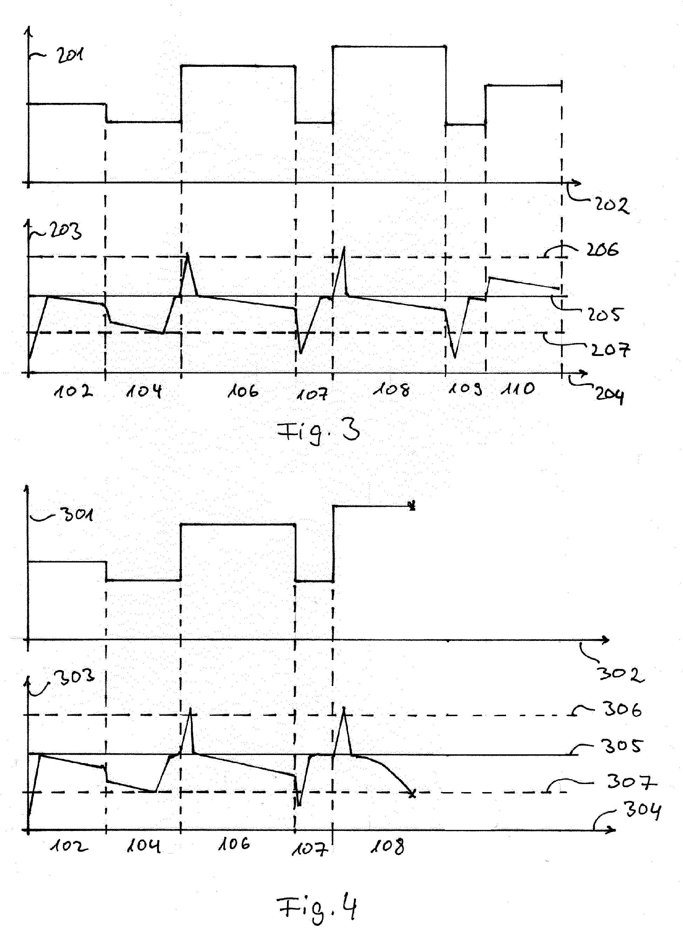

In der Figur 3 ist ein möglicher Verlauf einer Temperatur des Endoskops10 und des Drucks im Innenraum 13 des Endoskops dargestellt, welches durch den in Figur 2 dargestellten Prozess aufbereitet wird.

In dem oberen Diagramm ist auf der Hochachse 201 die Temperatur des Endoskops 10 in einem willkürlichen Maßstab dargestellt. Auf der Längsachse 202 ist die Zeit in einem willkürlichen Maßstab dargestellt.

Eine direkte Messung der Temperatur der Endoskops 10 während des Aufbereitungsvorgangs ist schwierig, da im Endoskop 10 hierzu in der Regel keine Sensoren vorgesehen sind. Die Temperatur entspricht aber recht genau der Temperatur der Spülflüssigkeit, welche beispielsweise durch den Sensor 9 gemessen werden kann. Anstelle einer Messung der Temperatur kann auch ein in der Steuerung 6 hinterlegter momentaner Sollwert der Temperatur verwendet werden.

In dem unteren Diagramm ist der Druck in dem Innenraum 13 des Endoskops auf der Hochachse 203 dargestellt, wiederum in willkürlicher Skalierung. Der Druck wird dabei durch Sensoren in dem Dichtigkeitstester 5 ermittelt. Eine durchgezogene waagerechte Linie 205 stellt einen Solldruck dar. Gestrichelte Linien 206, 207 stellen einen oberen und einen unteren Grenzwert für den Druck dar.

Auf der Längsachse 204 ist wiederum die Zeit dargestellt, wobei die Achsen 202 und 204 gleich skaliert sind. Die Prozessschritte des Aufbereitungsprozesses sind in den Diagrammen durch senkrechte Linien angedeutet. Das zu dem jeweiligen Prozessschritt gehörende Bezugszeichen ist zur besseren Orientierung unter der Achse 204 nochmals angegeben.

Im Prozessschritt 102 wird noch kein Spülmittel verwendet, die Temperatur entspricht daher der Umgebungstemperatur. Der Druck steigt zunächst von einem niedrigen Anfangsdruck auf den Solldruck an, während der Dichtigkeitstester 5 Luft in den Innenraum 13 des Endoskops 10 pumpt. Nachdem der Solldruck erreicht ist, fällt der Druck durch systemimmanente Undichtigkeiten langsam ab, ohne zunächst jedoch den unteren Grenzwert 207 zu erreichen.

Im Schritt 104 wird Wasser durch die Kanäle 11,12 des Endoskops 10 geleitet, dabei sinkt die Temperatur etwas ab. Dies hat einen entsprechenden Abfall des Drucks zur Folge, so dass sich der Druck dem unteren Grenzwert 207 annähert und diesen schließlich erreicht. Der Dichtigkeitstester 5 registriert den ersten Druckabfall unter den unteren Grenzwert 207. Wegen des vorhergehenden Temperaturabfalls wird der Druckabfall durch den Dichtigkeitstester 5 jedoch nicht als Defekt des Endoskops 10 interpretiert.

Anschließend pumpt der Dichtigkeitstester 5 weiter Luft in den Innenraum 13, so dass wieder der Solldruck 205 erreicht wird.

Im nächsten Prozessschritt 106 wird das Endoskop mit warmem Reinigungsmittel behandelt, so dass die Temperatur schnell ansteigt. Der Druck steigt ebenfalls bis auf den oberen Grenzwert 206 an, was den Dichtigkeitstester 105 veranlasst, Luft aus dem Innenraum 13 des Endoskops 10 abzulassen, bis der Duck wieder dem Solldruck 205 entspricht. Von dort fällt der Druck wieder langsam ab, ohne jedoch den unteren Grenzwert 207 zu unterschreiten.

Zu Beginn des Prozessschritts 107, in welchem das Endoskop 10 mit kaltem Wasser behandelt wird, fallen Temperatur und Druck stark ab, wobei der untere Grenzwert 207 des Drucks unterschritten wird. Auch dies wird von dem Dichtigkeitstester 5 nicht als Defekt des Endoskops 10 erkannt, da der Druckabfall mit einem Temperaturabfall einhergeht.

Der Dichtigkeitstester 5 pumpt wiederum Luft in den Innenraum 13 des Endoskops 10, bis der Solldruck 205 erreicht ist.

Im Schritt 108 erfolgt die Desinfektion des Endoskops 10, wobei die Temperatur noch höher ist als während des Schritts 106. Es erfolgt wieder ein Druckanstieg und ein Ablassen von Luft.

In Schritt 109 erfolgt ein Nachspülen des Endoskops 10 mit Wasser, so dass die Temperatur und der Druck wieder abfallen. Es wird ebenfalls der untere Grenzwert 207 unterschritten, aber das Endoskop 10 wird korrekt als intakt interpretiert.

Im Trocknungsschritt 110 steigt die Temperatur leicht an, dies hat aber keinen relevanten Einfluss mehr auf den Druck.

Es ist erkennbar, dass durch die Berücksichtigung der Temperatur bzw. des Temperaturverlaufs bei der Beurteilung des Druckverlaufs eine fehlerhafte Beurteilung des Endoskops 10 als defekt vermieden werden kann, ohne den unteren Grenzwert 207 für den Druck zu sehr niedrig ansetzen zu müssen, oder eine bestimmte Anzahl von Druckabfällen unter den Grenzwert unabhängig vom Temperaturverlauf zu erlauben. Dadurch wird die Erkennungsgüte des Dichtigkeitstesters 5 verbessert.

In Figur 4 ist der Verlauf des Drucks und der Temperatur während eines Aufbereitungsprozesses dargestellt, bei welchem ein Defekt des Endoskops 10 auftritt.

Die Achsen und Prozessschritte entsprechen denen der Figur 3. Sie sind daher mit einem jeweils um 100 erhöhten Bezugszeichen versehen und werden nicht erneut beschrieben.

In dem in Figur 4 dargestellten Aufbereitungsprozess kommt es im Prozessschritt 108, der Desinfektion bei hoher Temperatur, dazu, dass die Hülle des Endoskops 10 durch die Desinfektionslösung angegriffen wird. Dies kann beispielsweise eintreten, wenn das Endoskop 10 nach einer großen Zahl von Anwendungen und Aufbereitungsprozessen das Ende seiner Lebensdauer erreicht hat, oder wenn einer der Kanäle 11,12 während der Verwendung durch ein chirurgisches Instrument vorgeschädigt wurde.

Durch die Einwirkung der Desinfektionslösung wird die Hülle des Endoskops porös und Luftdurchlässig, so dass der Druck in dem Innenraum 13 des Endoskops vor dem Ende des Prozessschritts 108 den unteren Grenzwert 307 unterschreitet. Da dem Druckabfall kein entsprechender Temperaturabfall vorausgegangen ist, erkennt der Dichtigkeitstester 5 den Defekt des Endoskops 10 und bricht den Aufbereitungsprozess ab.

Das Endoskop 10 kann so einer Untersuchung und ggf. Reparatur zugeführt werden, bevor größere Mengen Desinfektionslösung in den Innenraum 13 eindringen und das Endoskop 10 dadurch ggf. irreparabel schädigen.

## Patentansprüche

1. Verfahren zum Aufbereiten eines Endoskops in einem Aufbereitungsgerät, mit den Schritten:
- Verbinden eines Innenraums (13) des Endoskops (10) mit einem Dichtigkeitstester (5) des Aufbereitungsgeräts (1),
- Beaufschlagen des Innenraums (13) mit einem Fluid unter einem vorgegebenen Druck, und
- Durchführen eines Aufbereitungsprozesses unter Einsatz von Spülflüssigkeit,
wobei der Druck in dem Innenraum (13) des Endoskops (10) während des Aufbereitungsprozesses überwacht wird, **dadurch gekennzeichnet, dass** das aufzubereitende Endoskop (10) als fehlerhaft erkannt wird, wenn der Verlauf des Drucks von einem vorgebbaren oder vorgegebenen Druckverlauf abweicht,
wobei der vorgegebene oder vorgebbare Druckverlauf in Abhängigkeit von Prozessparametern des Aufbereitungsprozesses festgelegt wird.

2. Verfahren nach Anspruch 1, **dadurch gekennzeichnet, dass** Fluid aus dem Innenraum (13) des Endoskops (10) abgelassen wird, wenn der Druck einen oberen Grenzwert übersteigt.

3. Verfahren nach Anspruch 1 oder 2, **dadurch gekennzeichnet, dass** zusätzliches Fluid in den Innenraum (13) des Endoskops (10) eingebracht wird, wenn der Druck einen unteren Grenzwert unterschreitet.

4. Verfahren nach Anspruch 3, **dadurch gekennzeichnet, dass** der vorgegebene oder vorgebbare Druckverlauf durch eine Zeit charakterisiert ist, welche maximal benötigt werden darf, um den Druck nach Unterschreiten des unteren Grenzwerts auf einen Sollwert zu bringen.

5. Verfahren nach Anspruch 3 oder 4, **dadurch gekennzeichnet, dass** der vorgegebene oder vorgebbare Druckverlauf durch eine maximale Häufigkeit charakterisiert ist, mit welcher der Druck den unteren Grenzwert unterschreiten darf.

6. Verfahren nach einem der Ansprüche 1 bis 5, **dadurch gekennzeichnet, dass** die Prozessparameter eine Temperatur der Spülflüssigkeit umfassen.

7. Verfahren nach einem der Ansprüche 1 bis 6, **dadurch gekennzeichnet, dass** die Prozessparameter den Verlauf der Temperatur der Spülflüssigkeit umfassen.

8. Verfahren nach Anspruch 6 oder 7, **dadurch gekennzeichnet, dass** die Temperatur bzw. der Temperaturverlauf der Spülflüssigkeit mittels in dem Aufbereitungsgerät vorgesehener Sensoren (9) bestimmt wird.

9. Verfahren nach Anspruch 6 oder 7, **dadurch gekennzeichnet, dass** die Temperatur bzw. der Temperaturverlauf der Spülflüssigkeit anhand einer in einer Steuerung (6) des Aufbereitungsgeräts (1) hinterlegten Solltemperatur bzw. eines Solltemperaturverlaufs bestimmt wird.

10. Verfahren nach einem der vorangehenden Ansprüche, **dadurch gekennzeichnet, dass**
- in der Steuerung (6) des Aufbereitungsgeräts (1) mehrere Aufbereitungsprogramme für unterschiedliche Endoskoptypen und/oder unterschiedliche Arten und/oder Intensitäten der Verschmutzung hinterlegt sind,
- zu Beginn der Aufbereitung des Endoskops (10) ein Aufbereitungsprogramm ausgewählt wird, und
- ein zu dem ausgewählten Aufbereitungsprogramm hinterlegter Temperaturverlauf der Spülflüssigkeit zur Festlegung des vorgegebenen oder vorgebbaren Druckverlaufs verwendet wird.

11. Aufbereitungsgerät für Endoskope, mit einem Aufbereitungsaggregat (4,7,8), einer Steuerung (6), und einem Dichtigkeitstester (5), **dadurch gekennzeichnet, dass** das Aufbereitungsgerät (1) eingerichtet ist, ein Verfahren gemäß eines der Ansprüche 1 bis 10 auszuführen.

## Claims

1. Method for reprocessing an endoscope in a reprocessing apparatus, comprising the steps:
- Connecting an interior space (13) of the endoscope (10) to a leakage tester (5) of the reprocessing apparatus (1),
- Applying a fluid with a predetermined pressure to the interior space (13), and
- Conducting a reprocessing process using rinsing fluid,
wherein the pressure in the interior space (13) of the endoscope (10) is monitored during the reprocessing process,
**characterized in that** the endoscope (10) to be reprocessed is recognized as defective if the pressure curve deviates from a predeterminable or predetermined pressure curve,
wherein the predeterminable or predetermined pressure curve is determined as a function of process parameters of the reprocessing process.

2. Method according to claim 1, **characterized in that** fluid is discharged from the interior space (13) of the endoscope (10) when the pressure exceeds an upper limit value.

3. Method according to claim 1 or 2, **characterized in that** additional fluid is introduced into the interior space (13) of the endoscope (10) when the pressure falls below a lower limit value.

4. Method according to claim 3, **characterized in that** the predetermined or predeterminable pressure curve is **characterized by** a maximum time which may be required to bring the pressure to a desired value after it has fallen below the lower limit value.

5. Method according to claim 3 or 4, **characterized in that** the predetermined or predeterminable pressure curve is **characterized by** a maximum number of times with which the pressure may fall below the lower limit value.

6. Method according to any one of claims 1 to 5, **characterized in that** the process parameters comprise a temperature of the rinsing liquid.

7. Method according to any one of claims 1 to 6, **characterized in that** the process parameters comprise the temperature curve of the rinsing liquid.

8. Method according to claim 6 or 7, **characterized in that** the temperature or the temperature curve of the rinsing liquid is determined by means of sensors (9) provided in the reprocessing device.

9. Method according to claim 6 or 7, **characterized in that** the temperature or the temperature curve of the rinsing liquid is determined on the basis of a target temperature or a target temperature curve stored in a controller (6) of the reprocessing device (1).

10. Method according to one of the preceding claims, **characterized in that**
- several reprocessing programs for different endoscope types and/or different types and/or intensities of contamination are stored in the controller (6) of the reprocessing device (1),
- a reprocessing program is selected at the beginning of the reprocessing of the endoscope (10), and
- a temperature curve of the rinsing liquid stored for the selected reprocessing program is used to determine the predefined or predeterminable pressure curve.

11. Reprocessing apparatus for endoscopes, having a reprocessing unit (4, 7, 8), a controller (6), and a leakage tester (5), **characterized in that** the reprocessing apparatus (1) is configured to conduct a method according to one of claims 1 to 10.

## Revendications

1. Procédé de traitement d'un endoscope dans un appareil de traitement, comprenant les étapes suivantes :
- Relier un espace intérieur (13) de l'endoscope (10) à un testeur d'étanchéité (5) de l'appareil de traitement (1),
- Appliquer sur l'espace intérieur (13) un fluide sous une pression prédéterminée, et
- Réaliser un procédé de traitement par liquide de rinçage,
dans lequel la pression à l'espace intérieur (13) de l'endoscope (10) est surveillée pendant l'opération de traitement,
**caractérisé en ce que** l'endoscope (10) à traiter est identifié comme défectueux si le profil de pression s'écarte d'un profil de pression prédéfinissable ou prédéfini,
dans lequel le profil de pression prédéfini ou prédéfinissable est défini en fonction de paramètres de procédé du procédé de traitement.

2. Procédé selon la revendication 1, **caractérisé en ce que** du fluide est évacué de l'intérieur (13) de l'endoscope (10) lorsque la pression dépasse une valeur limite supérieure.

3. Procédé selon la revendication 1 ou 2, **caractérisé en ce que** du fluide supplémentaire est introduit à l'intérieur (13) de l'endoscope (10) lorsque la pression descendre en dessous d'une valeur limite inférieure.

4. Procédé selon la revendication 3, **caractérisé en ce que** le profil de pression prédéfini ou prédéfinissable est **caractérisé par** un temps maximum qui peut être nécessaire pour amener la pression en dessous de la valeur limite inférieure jusqu'à une valeur de consigne.

5. Procédé selon la revendication 3 ou 4, **caractérisé en ce que** le profil de pression prédéfinissable ou prédéfini est **caractérisée par** une fréquence maximale avec laquelle la pression peut descendre en dessous de la valeur limite inférieure.

6. Procédé selon l'une quelconque des revendications 1 à 5, **caractérisé en ce que** les paramètres du procédé comprennent une température du liquide de rinçage.

7. Procédé selon l'une quelconque des revendications 1 à 6, **caractérisé en ce que** les paramètres du procédé comprennent le profil de la température du liquide de rinçage.

8. Procédé selon la revendication 6 ou 7, **caractérisé en ce que** la température ou le profil de température du liquide de rinçage est déterminé au moyen de capteurs (9) prévus dans l'appareil de traitement.

9. Procédé selon la revendication 6 ou 7, **caractérisé en ce que** la température ou le profil de température du liquide de rinçage est déterminé à l'aide d'une température de consigne ou d'un profil de température de consigne enregistré dans un régulateur (6) de l'appareil de traitement.

10. Procédé selon l'une quelconque des revendications précédentes, **caractérisé en ce que**
- plusieurs programmes de traitement sont enregistrés dans le régulateur (6) de l'appareil de traitement (1) pour différents types d'endoscopes et/ou différents types et/ou intensités de salissures,
- au début du traitement de l'endoscope (10), un programme de traitement est sélectionné, et
- un profil de température du liquide de rinçage, enregistré pour le programme de traitement sélectionné, est utilisé pour déterminer le profil de pression prédéfini ou pouvant être prédéfini.

11. Appareil de traitement pour endoscopes, comprenant un agrégat de traitement (4, 7, 8), un régulateur (6) et un testeur d'étanchéité (5), **caractérisé en ce que** l'appareil de traitement (1) est configuré pour réaliser un procédé selon l'une quelconque des revendications 1 à 10.
